# EUROPEAN PATENT SPECIFICATION

(11) **EP 2 818 173 B1**
(45) Date of publication and mention of the grant of the patent: **22.09.2021**
(21) Application number: 13752335.3
(22) Date of filing: 22.02.2013
(51) Int. Cl.: A61K 35/12, A61L 27/00, A61P 19/02, A61P 19/04, A61P 19/08, A61P 21/00, A61P 37/00, A61L 27/38

(54) **CELL PREPARATION INCLUDING FAT CELL**
ZELLPRÄPARAT MIT FETTZELLEN
PRÉPARATION CELLULAIRE INCLUANT UNE CELLULE ADIPEUSE

(30) Priority: 24.02.2012 JP 2012038323
(43) Date of publication of application: 31.12.2014
(73) Proprietor: Saeki, Masanori, Tamano-shi, Okayama 706-0224 (JP)
(72) Inventor: Saeki, Masanori, Tamano-shi, Okayama 706-0224 (JP)
(74) Representative: Mewburn Ellis LLP
(86) International application number: PCT/JP2013/054483
(87) International publication number: WO 2013/125674

(56) References cited:
- JP-A- 2005 519 883
- JP-A- 2007 525 422
- JP-A- 2009 501 526
- US-A1- 2010 028 310
- US-A1- 2011 158 968
- US-A1- 2011 293 577
- SINEAD P BLABER ET AL: "Analysis of in vitro secretion profiles from adipose-derived cell populations", JOURNAL OF TRANSLATIONAL MEDICINE, vol. 10, no. 1, 1 January 2012 (2012-01-01), page 172, XP055054274, ISSN: 1479-5876, DOI: 10.1016/S1474-4422(02)00040-6
- S. YAMASAKI: 'Cytokines regulate fibroblast-like synovial cell differentiation to adipocyte-like cells' RHEUMATOLOGY vol. 43, 2004, pages 448 - 452, XP055082079
- B. SEYOUM, A. FITE, A.B. ABOU-SAMRA: 'Effects of 3T3 adipocytes on interleukin-6 expression and insulin signaling in L6 skeletal muscle cells' BIOCHEM. BIOPHYS. RES. COMMUN. vol. 410, no. 1, 2011, pages 13 - 18, XP028231223
- BERNAT BAEZA-RAJA AND PURA MUñOZ-CáNOVES: 'p38 MAPK-induced Nuclear Factor-?B Activity Is Required for Skeletal Muscle Differentiation: Role of Interleukin-6' MOL. BIOL. CELL vol. 15, April 2004, pages 2013 - 2026, XP002505646
- AARON W. B. JOE, ET AL.: 'Muscle injury activates resident fibro/adipogenic progenitors that facilitate myogenesis, (+Methods online and Supplementary Information)' NAT. CELL BIOL. vol. 12, February 2010, pages 153 - 163+16 PP, XP055082083
- None

## Description

### TECHNICAL FIELD

The present invention relates to a cell preparation for use in the treatment of osteoarthritis. In addition, the present invention relates to a cell preparation for use in the restoration of a degenerated or damaged muscle.

### BACKGROUND ART

As a means for increasing the volume of a soft tissue of human being, injection of hyaluronic acid, transplantation of a fat cell, and the like are known, and are frequently used in the field of cosmetic surgery, plastic surgery and the like such as removal of face wrinkle, excavation treatment and breast reconstruction. However, while treatment by injection of hyaluronic acid is easy, there is a problem that the injected hyaluronic acid is absorbed in about a half year, and that thus durability of the effect is poor. In addition, also as for transplantation of a fat cell, the fat cell sometimes necroses or is absorbed in a living body after transplantation. Thus, there is a disadvantage that reoperation may be required or scar may occur in some cases.

In order to eliminate these disadvantages, transplantation of an adipose stem cell was proposed, and a method for efficiently separating an adipose stem cell has been developed (for example, see Patent Documents 1 and 2). By transplanting an adipose stem cell, fixation ratio of the cell after transplantation is improved, and a certain effect is recognized in regeneration of a soft tissue. However, the adipose stem cell obtained by such a method may include impurities in some cases, and there has been a possibility that the impurities cause necrosis of a fat cell or a lump (calcification) after transplantation. A fat cell is generally obtained by liposuction, and the sucked adipose is a mixture including a wide variety of impurities in addition to a fat cell and an adipose stem cell. Impurities which should be removed from such a mixture include blood (especially erythrocytes), fatal/vital cells, senescent cells and the like. On the other hand, in Patent Document 3 is proposed a method of injecting a cell preparation (Condensed Rich Fat: CRF (registered trademark)) including a healthy concentrated fat cell and an adipose stem cell prepared by removing impurities which cause calcification or fat necrosis from a mixture collected by liposuction including a fat cell and an adipose stem cell by centrifugation using a syringe equipped with a weight filter. By such a method, fixation ratio of the cells is improved, and a problem such as a lump hardly occurs. Thus, such a method is becoming widely accepted.

As described above, a wide variety of techniques for separation and concentration of a fat cell and an adipose stem cell are developed, and a fat cell and an adipose stem cell which are safe and excellent in fixability are coming to be provided. However, transplantation of a fat cell and an adipose stem cell has been applied to regeneration of a soft tissue in most cases so far, and efficacy of these cells in regard to, for example, alleviation of arthralgia or regeneration of the tissue in a joint has not been sufficiently examined. In addition, it has not been known that a muscle is restored by administering a fat cell and an adipose stem cell to a damaged muscle.

Patent document 4 concerns the use of an adipose tissue cell suspensions comprising fat filled adipocytes, for use in the treatment of an inflammatory disorders, a cartilage or bone disorder and the pain associated with the inflammatory disorder.

Patent document 5 concerns the removal of adipose tissue from a subject, processing of the adipose tissue to increase the a concentration of adipose stem cells, mixing the concentrated suspension with a portion of adipose tissue and administering the resulting suspension to a subject for use in the treatment of bone-related disorders, skeletal muscle disorders, and for cartilage and joint repair.

Patent document 6 concerns the transplantation of adipose stem cells and/or fat cells and a semi-solid substance such as hyaluronic acid, collagen, thrombin, elastin chondroitin sulfate and/or albumin.

### PRIOR ART DOCUMENTS

### PATENT DOCUMENTS

Patent Document 1: JP 2005-519883 W
Patent Document 2: JP 2007-509601 W
Patent Document 3: JP 2007-533396 W
Patent Document 4: US 2011/293577 A1
Patent Document 5: US 2011/128968 A1
Patent Document 6: US 2010/028310 A1

### SUMMARY OF THE INVENTION

### PROBLEMS TO BE SOLVED BY THE INVENTION

A main object of the present invention is to provide a cell preparation for use in the treatment of osteoarthritis. In addition, another object of the present invention is to provide a cell preparation for use in the restoration of a damaged muscle.

### SOLUTIONS TO THE PROBLEMS

The present inventor made earnest investigations for solving the above-mentioned problems and found that injection of a combination of a micronized fat cell and an adipose stem cell results in efficient regeneration of a bone tissue or a cartilage tissue in the joint in addition to the above-mentioned alleviation of arthralgia. In addition, the present inventor found that a damaged muscle is restored by administering a cell preparation including a micronized fat cell and an adipose stem cell to a site of muscle damage. As a result of studies further continued on the basis of these findings, the present invention was completed.

A first aspect of the invention is a cell preparation for use in the treatment of osteoarthritis, comprising:
a mixture including a micronized fat cell and an adipose stem cell, the micronized fat cell and the adipose stem cell being obtained by steps of:
separating an adipose tissue taken from a subject into a cellular fraction and a liquid fraction to obtain the cellular fraction;
removing impurities from the cellular fraction to obtain a concentrate of a fat cell and an adipose stem cell; and
micronizing the fat cell in the concentrate of the fat cell and the adipose stem cell, wherein there are 100,000 or more adipose stem cells per 1 mL of the cell preparation.

A second aspect of the invention is a cell preparation for use in the treatment of osteoarthritis, comprising:
a mixture including a micronized fat cell and an adipose stem cell, wherein the micronized fat cell and the adipose stem cell are obtained by steps of:
separating an adipose tissue taken from a subject into a cellular fraction and a liquid fraction to obtain the cellular fraction;
removing impurities from the cellular fraction to obtain a concentrate of a fat cell and an adipose stem cell;
micronizing the fat cell in the concentrate of the fat cell and the adipose stem cell to obtain a mixture including the micronized fat cell and the adipose stem cell;
separating the adipose stem cell from the mixture of the micronized fat cell and the adipose stem cell to obtain the adipose stem cell; and
mixing the separated adipose stem cell with the concentrate of the micronized fat cell and the adipose stem cell, wherein there are 100,000 or more adipose stem cells per 1 mL of the cell preparation.

In some embodiments, the cell preparation for use in the treatment of osteoarthritis according to the first or second aspects, comprising 1 to 10 adipose stem cells per one fat cell.

A third aspect of the invention is a method for manufacturing a cell preparation for use in the treatment of osteoarthritis, comprising steps of:
separating an adipose tissue taken from a subject into a cellular fraction and a liquid fraction to obtain the cellular fraction;
removing impurities from the cellular fraction to obtain a concentrate of a fat cell and an adipose stem cell; and
micronizing the fat cell in the concentrate of the fat cell and the adipose stem cell to obtain a mixture including the micronized fat cell and the adipose stem cell, wherein
the cell preparation comprises a mixture including a micronized fat cell and an adipose stem cell, wherein there are 100,000 or more adipose stem cells per 1 mL of the cell preparation.

A fourth aspect of the invention is a method for manufacturing a cell preparation for use in the treatment of osteoarthritis, comprising steps of:
separating an adipose tissue taken from a subject into a cellular fraction and a liquid fraction to obtain the cellular fraction;
removing impurities from the cellular fraction to obtain a concentrate of a fat cell and an adipose stem cell;
micronizing the fat cell in the concentrate of the fat cell and the adipose stem cell to obtain a mixture including the micronized fat cell and the adipose stem cell;
separating the adipose stem cell from the mixture including the micronized fat cell and the adipose stem cell to obtain the adipose stem cell; and
mixing the separated adipose stem cell with remainder of the concentrate of the micronized fat cell and the adipose stem cell to obtain the mixture including the micronized fat cell and the adipose stem cell, wherein
the cell preparation comprises a mixture including a micronized fat cell and an adipose stem cell, wherein there are 100,000 or more adipose stem cells per 1 mL of the cell preparation.

In some embodiments, the method for manufacturing the cell preparation for use in the treatment of osteoarthritis according to the third or fourth aspect, wherein the cell preparation comprises 1 to 10 adipose stem cells per one fat cell.

A fifth aspect of the invention is a cell preparation for use in the restoration of a muscle, comprising a mixture including a micronized fat cell and an adipose stem cell, wherein the micronized fat cell and the adipose step cell are obtained by the steps of:
separating an adipose tissue taken from a subject into a cellular fraction and a liquid fraction to obtain the cellular fraction;
removing impurities from the cellular fraction to obtain a concentrate of a fat cell and an adipose stem cell; and
micronizing the fat cell in the concentrate of the fat cell and the adipose stem cell, wherein there are 100,000 or more adipose stem cells per 1 mL of the cell preparation.

A sixth aspect of the invention is a method for manufacturing a cell preparation for use in the restoration of a muscle, the method comprising steps of:
separating an adipose tissue taken from a subject into a cellular fraction and a liquid fraction to obtain the cellular fraction;
removing impurities from the cellular fraction to obtain a concentrate of a fat cell and an adipose stem cell; and
micronizing the fat cell in the concentrate of the fat cell and the adipose stem cell to obtain a mixture including the micronized fat cell and the adipose stem cell, wherein
the cell preparation comprises the mixture including the micronized fat cell and the adipose stem cell, wherein there are 100,000 or more adipose stem cells per 1 mL of the cell preparation.

### EFFECTS OF THE INVENTION

In addition, according to the cell preparation including a micronized fat cell and an adipose stem cell of the present invention, pain caused by osteoarthritis is alleviated by the fat cell, during which time regeneration of a tissue in the joint such as a bone tissue, a cartilage tissue or a soft tissue by the mesodermal stem cell is enhanced. In addition, by using a combination of a micronized fat cell and an adipose stem cell, not only the above-mentioned pain can be alleviated, but also take percentage of the adipose stem cell and efficiency of regeneration of the tissue can be still more enhanced, since the adipose stem cell is retained in the affected area by the action of the micronized fat cell. Thus, the cell preparation of the present invention including a micronized fat cell and an adipose stem cell is further effective for treatment of osteoarthritis.

In addition, the cell preparation of the present invention including a micronized fat cell and an adipose stem cell has an excellent restoration effect on a muscle damaged by degeneration of the muscle or excision of the muscle by surgery. In addition, according to the cell preparation, pain caused by muscle damage can be early alleviated, and thus the cell preparation also contributes to improvement of QOL (Quality of Life) of the patient.

Conventionally, when a method of restoring a tissue by culturing a mesodermal stem cell such as an adipose stem cell is employed, there has been a possibility of contamination in the course of culturing and the like, and an infectious disease could have been caused after transplantation. However, since the micronized fat cell and the adipose stem cell included in the cell preparation of the present invention can be directly separated and concentrated from the sucked adipose or the like without undergoing a step of cell culturing, the micronized fat cell and the adipose stem cell can be prepared without contacting with the open air. Therefore, there is no danger of an infectious disease or the like. In addition, since the cell preparation of the present invention is generally prepared using a micronized fat cell and an adipose stem cell collected from a patient himself/herself who requires treatment of osteoarthritis or restoration of a muscle, the cell preparation has no problem of immunorejection and is highly safe.

### BRIEF DESCRIPTION OF THE DRAWINGS

Fig. 1(a) shows an X-ray photograph showing the left knee joint of subject A before administration of a cell preparation, and Fig. 1(b) shows an X-ray photograph showing the left knee joint of subject A about 1 month after administration of the cell preparation.
Fig. 2(a) shows an X-ray photograph showing the right and left knee joints of subject B before administration of a cell preparation, and Fig. 2(b) shows an X-ray photograph showing the right and left knee joints of subject B about 1 month after administration of the cell preparation.
Fig. 3 shows a representative example of MR images showing that regeneration of a bone tissue was enhanced and osteonecrosis was improved or disappeared by administering a cell preparation.

### EMBODIMENTS OF THE INVENTION

### 1. Cell preparation

The present invention provides the cell preparation for use in the treatment of osteoarthritis and for restoration of a muscle described below. The cell preparations will be described in detail herein below.

### (1-1) Cell preparation for use in the treatment of osteoarthritis

A fat cell is a cell which has functions of synthesizing, storing and releasing fat and which forms an adipose tissue. In addition, since a fat cell has been completely differentiated, the cell does not undergo cell division. The fat cell is classified into a white fat cell and a brown fat cell. Either of the cells may be used in the present invention, or a mixture of both the cells may be used. In the present invention, the origin of the fat cell is not specifically limited, and, for example, a fat cell of a mammal, preferably a fat cell of a primate, further preferably a human fat cell is used. In the present invention, as a fat cell, a fat cell alone may be used, or a mixture of an extracellular matrix (ECM) existing around a fat cell and a fat cell may be used. Specifically, in the present invention, a collected fat cell may be used as it is, or a fat cell prepared by removing the extracellular matrix from the collected fat cell may be used.

A fat cell is collected from an adipose tissue. A method for collecting an adipose tissue is not specifically limited, and appropriately selected from conventionally known methods, including, for example, liposuction, lipectomy, and defatting. Collection by liposuction is preferable, since liposuction is relatively easy and minimally invasive. A method for obtaining a fat cell from the collected fat is not specifically limited, and is preferably the method of 2. Method for preparing a cell preparation described below.

In addition, the cell preparation for use in the treatment of osteoarthritis of the present invention further includes an adipose stem cell. By including an adipose stem cell, regeneration of a tissue in the interosseous joint is enhanced, and the therapeutic effect by the cell preparation of the present invention is still more increased.

An adipose stem cell can be collected from an adipose tissue. The adipose tissue used as the source of the adipose stem cell can also be collected by any of the methods such as liposuction, lipectomy and defatting as with the above-mentioned fat cell, and the method is preferably liposuction from the viewpoint of preventing outflow or damage of the adipose stem cell and the viewpoint of safer collection. In addition, a preferable example of a method for obtaining an adipose stem cell from the collected fat is the method described in 2. Method for preparing a cell preparation described below.

In a case of a cell preparation prepared by the method described below including the steps of (1) and (2), the number of the adipose stem cells included in 1 mL of the cell preparation is 100 thousand or more, preferably 150 thousand or more, further preferably 150 to 200 thousand.

In the cell preparation of the present invention, by using an autologous fat cell and an autologous adipose stem cell for the purpose of autologous transplantation, immunorejection can be avoided in addition to enablement of early improvement of the symptom. However, preparation of the cell preparation of the present invention using a fat cell and an adipose stem cell derived from another person is not limited by the above. When using a cell derived from another person, it is preferable to carry out treatment for removing antigenicity such as, for example, gamma irradiation for the fat cell (including the extracellular matrix (ECM) existing around the fat cell) collected from another person before transplantation. While the adipose stem cell is a cell generally having no immunogenicity, an adipose stem cell which has been subjected to the above-mentioned treatment may also be used, if needed.

Generally, an adipose tissue includes a fat cell and an adipose stem cell. Thus, the cell preparation including a micronized fat cell and an adipose stem cell, is prepared by removing impurities (for example, an anesthetic solution injected upon collection of fat (so-called tumescent fluid), an aged fat cell, blood, and tissue fluid) from the collected adipose tissue, a cell preparation prepared by removing the impurities and a part of a fat cell from the collected adipose tissue, and the like may be used.

In addition, a conventionally known pharmaceutically acceptable carrier, excipient, antiphlogistic, analgesic, immunosuppressive or the like can be added to the cell preparation of the present invention, if needed. In addition, collagen, a fibroblast, a growth factor, a proliferator, a cytokine or the like may be added thereto so long as the effect of the present invention is not negated.

In addition, for example, another cell preparation of which action of regenerating a tissue is known such as PRP (Platelet Rich Plasma) may be used in combination with the cell preparation of the present invention. Furthermore, by adding an angiotensin II receptor antagonist such as losartan to PRP, the effect of regeneration of a cartilage tissue can be still more augmented. However, from the viewpoint of improving the efficiency of regeneration of a tissue, for example, it is preferable to remove VEGFs (vascular endothelial growth factors) from PRP before use in a case where regeneration of a cartilage tissue is expected, and it is preferable to remove BMPs (bone morphogenetic proteins) before use in a case where bone regeneration is expected.

The cell preparation for use in the treatment of osteoarthritis of the present invention exhibits an excellent therapeutic effect for osteoarthritis.

The dose of the cell preparation of the present invention is not specifically limited so long as the dose can give a therapeutic effect for osteoarthritis, and can be appropriately set depending on the size of the part of administration, the site of the disease, the degree of the disease, and sex, physique and the like of the patient. For example, the dose is 100 thousand to 4 million cells, preferably 200 thousand to 2 million cells, further preferably 200 thousand to 1 million cells as converted into the number of the fat cells. For example, when a cell preparation obtained by the method described below including the steps of (1) and (2) is used, the dose is 0.5 to 20 mL, preferably 1 to 10 mL, further preferably 1 to 5 mL. In addition, for example, regarding the knee joint, 1 to 20 mL, preferably 1 to 10 mL, further preferably 5 to 10 mL of the cell preparation can be administered. In addition, regarding the intervertebral disk, 0.5 to 20 mL, preferably 1 to 15 mL, further preferably 1 to 2 mL of the cell preparation can be administered.

Examples of the administration method include, but not limited to, a method of injection to the part of the disease of an interosseous joint (for example, joint space and intervertebral disk (intervertebral disc)) using a syringe, a cannula or the like. In addition, upon injection, a still more excellent therapeutic effect can be obtained by stimulating the portion of injection with the tip of a needle or a cannula. Examples of the method of stimulation include, but not specifically limited to, a method of stimulating the site of injection by turning a cannula of which tip is obliquely cut in all directions upon injecting the cell preparation.

When the cell preparation of the present invention is administered to patients having osteoarthritis, the cell preparation may be administered after carrying out a treatment by a known method, if needed.

### (1-2) Cell preparation for restoration of a muscle

The present invention provides a cell preparation capable of restoring a muscle in a site of muscle damage, characterized in that the cell preparation includes a micronized fat cell and an adipose stem cell.

The micronized fat cell and the adipose stem cell used in the cell preparation for restoration of a muscle of the present invention, and the content of both the cells in the cell preparation and the like are as described in the section of "(1-1) Cell preparation for use in the treatment of osteoarthritis". . Examples of the method for obtaining an adipose stem cell include the method described in 2. Method for preparing a cell preparation described below.

In addition, also in the cell preparation for restoration of a muscle, a conventionally known pharmaceutically acceptable carrier, excipient, antiphlogistic, analgesic, immunosuppressive or the like can be added, if needed, in addition to the fat cell and the mesodermal stem cell. In addition, collagen, a fibroblast, a growth factor, a proliferator, a cytokine or the like may be added thereto so long as the effect of the present invention is not negated.

In addition, for example, another cell preparation of which action of regenerating a tissue is known such as PRP (Platelet Rich Plasma) may be used in combination with the cell preparation of the present invention so long as the effect of the present invention is not negated. However, from the viewpoint of still more improving the efficiency of regeneration of a muscle tissue, it is preferable to remove transforming growth factor β1 (TGF β1) before use.

The cell preparation for restoration of a muscle of the present invention exhibits an excellent effect of restoration of a muscle when applied to a site of muscle damage. Muscle damage includes a case in which a muscle has been excised by surgery, injury or the like, in addition to myodegeneration, myofibrosis, destruction or necrosis of a muscle fiber, amyotrophy, muscular paralysis, muscle weakness and the like. In addition, examples of the site or kind of muscle to which the cell preparation of the present invention is applied include, but not specifically limited to, skeletal muscles such as trunk muscles (including a muscle of the upper or lower limb and pectoralis major); smooth muscles such as bladder sphincter; and a myocardium. For example, by administering the cell preparation of the present invention to a site of damage of the muscle of the upper limb, pain due to muscle damage can be early alleviated and the muscle is restored, thereby expansion of ROM (Range of Motion) or improvement in motor function of the upper limb can be realized. In addition, by administering the cell preparation of the present invention to the bladder sphincter of a patient presenting urination disorder such as urinary incontinence or frequent urination due to muscle weakness, damage or the like of bladder sphincter, a problem of urinary incontinence, incontinence or the like can be safely and easily eliminated by increasing muscle strength of the bladder sphincter. In addition, the cell preparation of the present invention can also be applied to the site of muscle damage of a patient having a muscular disease such as myocardial infarction, ischemia-reperfusion injury, muscular dystrophy (for example, Duchenne's muscular dystrophy (DMD)) or myositis (myopathy).

In addition, muscle damage is often painful, but the pain can be early alleviated by administering the cell preparation for restoration of a muscle of the present invention to the site of muscle damage. Without expecting limitative interpretation of the present invention, it is thought that such effect of alleviating pain is due to the fat cell included in the cell preparation for restoration of a muscle.

The dose of the cell preparation of the present invention when used for the purpose of restoration of a muscle is not specifically limited so long as the dose can give an effect of restoration of a muscle, and can be appropriately set depending on the size of the part of administration, the site of the disease, the degree of the disease, and sex, physique and the like of the patient. For example, the dose per unit volume of 10 mL of damaged muscle is 100 thousand to 4 million cells, preferably 200 thousand to 2 million cells, further preferably 200 thousand to 1 million cells as converted into the number of the fat cells. In addition, when a cell preparation obtained by the method described below including the steps of (1) and (2) is used, the dose per unit volume of 10 mL of damaged muscle is 0.5 to 20 mL, preferably 1 to 10 mL, further preferably 1 to 5 mL.

The administration method of the cell preparation of the present invention when used for the purpose of restoration of a muscle is not specifically limited, and a conventionally known administration method can be employed. For example, a method of injection to the site of muscle damage with a syringe, a cannula or the like can be mentioned.

### 2. Method for preparing a cell preparation

In the present invention, when preparing a cell preparation including a micronized fat cell and an adiposestem cell, a fat cell collected as a mixture of these cells is used. For example, the adipose stem cell is simultaneously prepared from the same tissue as the fat cellto obtain a mixture of a fat cell and an adipose stem cell from an adipose tissue to give the cell preparation of the present invention. In addition, more easily, an adipose tissue including these cells may be used.

When preparing the cell preparation of the present invention from an adipose tissue, the method described below can be mentioned as a preferable method. However, this method does not limit preparation of the cell preparation of the present invention from a tissue other than fat, and the cell preparation of the present invention can also be prepared by appropriately setting conditions, considering the kind or properties of the cell according to the method described below.

The method for preparing the cell preparation of the present invention includes a step (1) of removing a liquid fraction from the collected fat to give a cellular fraction.

Here, the collected fat indicates a mixture of a cellular fraction and a liquid fraction derived from an adipose tissue that is collected from an adipose tissue by liposuction, lipectomy, defatting or the like. The cellular fraction includes a fat cell, an adipose stem cell and impurities (hemocytes, fatal/vital cells, senescent cells and the like). In addition, the liquid fraction includes a tumescent fluid, a cellular tissue fluid or the like.

Separation of the liquid fraction from the cellular fraction can be easily carried out by precipitating the cellular fraction by leaving the collected fat standing still. Examples of the method for removing the liquid fraction include, but not specifically limited to, decantation and suction. In addition, centrifugation, filtration or the like can be carried out, if needed.

Conditions for centrifugation and filtration are not specifically limited so long as the fat cell and the adipose stem cell are not damaged and the impurities can be removed. Examples of the conditions include the conditions of at 700 to 2,500 × g for 1 to 15 minutes, preferably the conditions of at 2,000 to 2,200 × g for 5 to 10 minutes.

In addition, when filtration is carried out, for example, blood, tissue fluid, anesthetic administered to the part from which the fat has been collected (a tumescent fluid) and the like are removed by letting the collected fat through a mesh filter with pores having a size of 10 to 300 µm, preferably 10 to 150 µm, more preferably 10 to 100 µm, further preferably 15 to 20 µm to give a mixture of a fat cell and an adipose stem cell on the filter. In addition, a container equipped with such a mesh filter may be charged with the collected fat containing impurities, and the fat may be subjected to centrifugation.

Thus, an adipose tissue including a fat cell (including an extracellular matrix) and an adipose stem cell from which a liquid fraction has been removed can be obtained, and the adipose tissue can be used as the cell preparation of the present invention.

The method for preparing a cell preparation of the present invention includes a step (2) of removing impurities from the cellular fraction obtained in step (1) to give a concentrate of a fat cell and an adipose stem cell.

In step (2), the cellular fraction is concentrated by removing impurities therefrom. The method for removing impurities and the method for concentration are not specifically limited, and can be appropriately selected from the conventionally known methods. As an easy method, for example, by using the syringe equipped with a weight filter described in Patent Document 3, impurities such as free oil released from the broken fat cell, fatal/vital cells and senescent cells are removed to give a concentrate of a healthy fat cell and an adipose stem cell. Such a syringe is commercially available, and a specific example thereof is a syringe manufactured for LIPOMAX-SC (manufactured by Medikan International Inc., Seoul, Korea).

More specifically, a syringe equipped with a filter therein is charged with the collected fat, and the fat is separated into a liquid fraction and a cellular fraction by centrifugation. Furthermore, free oil (drainage oil) is separated by the filter. The liquid fraction separated here is a liquid which has not been completely separated from the cellular fraction and mixed therewith in step (1). That is to say, in the syringe, free oil, a concentrate of a fat cell and an adipose stem cell, and a liquid fraction are separated in the upper layer, the intermediate layer and the lower layer, respectively. By discarding the layers other than the intermediate layer, a concentrate of a healthy fat cell and an adipose stem cell can be obtained, and the concentrate can be used as the cell preparation of the present invention. Since a healthy fat cell and an adipose stem cell are selected and concentrated, the cell preparation thus obtained hardly causes necrosis, calcification or the like after transplantation, and is still more useful in alleviation and treatment of symptoms of osteoarthritis, restoration of a damaged muscle and alleviation of pain due to muscle damage.

Conditions for centrifugation for separating the fat into a liquid fraction and a cellular fraction are not specifically limited so long as the fat cell and the adipose stem cell are not damaged and the impurities are separated from the fat cell and the adipose stem cell. Examples of the conditions include the conditions of at 700 to 2,500 × g for 1 to 15 minutes, preferably the conditions of at 2,000 to 2,200 × g for 5 to 10 minutes.

In addition, the pore size of the filter is not specifically limited so long as a fat cell and an adipose stem cell cannot pass through the pore and impurities can pass through the pore. The size is, for example, 10 to 300 µm, preferably 10 to 150 µm, more preferably 10 to 100 µm, further preferably 15 to 20 µm.

In addition, the present method may include a step (3') of separating a fat cell and an adipose stem cell from at least a part of the concentrate obtained in step (2) and further mixing the obtained adipose stem cell with the concentrate obtained in step (2).

Here, examples of the method for separating an adipose stem cell include the method described in Patent Document 1. More specifically, in order to facilitate separation of a fat cell and an adipose stem cell by degrading intercellular junction, a protease is added to the cellular fraction. Examples of the protease include collagenase, trypsin, and lipase. One kind of the protease selected therefrom may be used alone, or two or more kinds of the proteases may be used in combination.

The method for separating a fat cell and an adipose stem cell from the cellular fraction treated with an enzyme is not specifically limited, and can be appropriately selected from the conventionally known methods. For example, the method may be centrifugation, filtration or the like. Conditions for centrifugation are not specifically limited so long as the fat cell and the adipose stem cell can be separated without damaging the cells. Examples of the conditions include the conditions of at 100 to 500 × g for 1 to 20 minutes, preferably the conditions of at 100 to 300 × g for 5 to 10 minutes. By subjecting the cellular fraction to such centrifugation, an adipose stem cell can be obtained as a precipitate, and a fat cell is concentrated in the supernatant. In addition, when filtration is carried out, an adipose stem cell can be obtained on a filter by letting the cellular fraction or a suspension thereof through a mesh filter with pores having a size of 10 to 100 µm, preferably 10 to 50 µm, further preferably 15 to 20 µm, and a fat cell can be obtained in the filtrate.

The fat cell and the adipose stem cell treated with an enzyme is preferably subjected to washing treatment after separation, and washing can be carried out with phosphate buffered saline (PBS), saline, Ringer's solution, dextran or the like. The washing treatment may be repeated multiple times if needed, or the cells may be subjected to centrifugation if needed, upon collecting the cells after washing. The conditions for centrifugation can follow the conditions employed upon separating a fat cell and an adipose stem cell from the cellular fraction treated with an enzyme.

In the present step (3'), when an adipose stem cell is separated using a protease, generally about 700 thousand or more, preferably about 1.5 million or more, further preferably about 2 million or more of an adipose stem cell per 1 cc of the concentrate obtained in step (2) can be obtained. Since a healthy fat cell is selected and concentrated, and moreover an adipose stem cell is included in a higher concentration, the cell preparation prepared via the present step (3') exhibits a still more excellent effect on treatment of osteoarthritis and restoration of a damaged muscle.

In addition, the fat cell separated in the present step (3') can be washed, and thereafter used as the cell preparation of the present invention. Since a healthy fat cell is selected and concentrated, the fat cell obtained here exhibits a still more excellent effect in alleviation of osteoarthritis or pain caused by muscle damage.

In addition, the present method includes a step (3") of micronizing the fat cell in the obtained concentrate after step (2), and separating the micronized fat cell and an adipose stem cell as a mixture. Micronization of the fat cell can be carried out by a method generally employed in the art, and is not specifically limited. For example, a fat cell is cut with a drill or the like if needed, and thereafter a mixture of the micronized fat cell and an adipose stem cell is separated. As the method for separation, the above-mentioned conditions for centrifugation can be employed. Upon carrying out the present step (3"), after step (2), the concentrate may be subjected to micronization and centrifugation of a fat cell while the concentrate is remaining in the syringe. Since free oil is separated from the mixture of the micronized fat cell and an adipose stem cell after centrifugation, the free oil can be discarded to give a mixture of a micronized fat cell and an adipose stem cell.

By undergoing such a separation step, a cell preparation including a finer fat cell and an adipose stem cell in a high concentration can be prepared. Here, a fine fat cell indicates a fat cell having a size such that it can pass through a needle of which gauge is 26 to 30, preferably 18 to 30. In the present step (3"), a treatment to micronize the fat cell can be carried out at least once, preferably once to several times, more preferably once to twice. By micronizing the fat cell, the concentration of the adipose stem cell per unit volume of the cell preparation can be increased, and the effect of alleviating osteoarthritis or pain derived from muscle damage, the effect of regeneration of a tissue inside an interosseous joint and the effect of restoration of a muscle can be exhibited still more remarkably.

In addition, a step (4") of separating an adipose stem cell from at least a part of the mixture of a micronized fat cell and an adipose stem cell obtained in step (3") and mixing the obtained adipose stem cell with the concentrate obtained in step (2) may be included. The method for separating an adipose stem cell is as described in regard to step (3').

The fat cell obtained by separating the adipose stem cell in the present step (4") can be washed, and thereafter used as a cell preparation including a fat cell of the present invention. Washing can be carried out by the method same as the above-mentioned washing treatment carried out for the adipose stem cell. Since a healthy, micronized fat cell is selected and concentrated, the fat cell obtained here exhibits a further remarkably excellent effect in alleviation of pain caused by osteoarthritis or muscle damage.

In the present method, by using the syringe described in Patent Document 3, all of the steps can be carried out without contacting the cells with the open air. For example, a cannula or the like may be attached to the syringe described in Patent Document 3, the syringe may be charged with the fat collected by liposuction, and the fat can be subjected to removal of impurities and a concentration treatment as it is to give a cell preparation. Thereafter, also upon administering the cell preparation, since the cell preparation can be directly transferred from the syringe to a syringe for injecting the cell preparation and administered to the affected part, a cell preparation having no problem such as contamination and high safety can be obtained. As an easy method, the present method can be carried out using a commercially available kit, and a specific example of such a kit is LIPOMAX-SC (manufactured by Medikan International Inc.).

The cell preparation of the present invention can be obtained by the above-mentioned method. Since impurities have been removed and a micronized, healthy fat cell is included in a high concentration, the cell preparation including the micronized fat cell obtained via step (4") of the method exhibits a remarkable effect on treatment of osteoarthritis (especially alleviation of pain). The cell preparation including a micronized fat cell and an adipose stem cell obtained via step (3") of the method includes an adipose stem cell in a high concentration, in addition to a micronized, healthy fat cell. Such a cell preparation can exhibit a further remarkably excellent effect on treatment of osteoarthritis and restoration of a muscle.

In the cell preparation including a micronized fat cell and an adipose stem cell for use in the treatment of osteoarthritis the method for collecting fat, the method for separating a mesodermal stem cell, the method for preparing a cell preparation, the method for administration to a patient and the like are as described in the section of "1. Cell preparation" and "2. Method for preparing a cell preparation".In the cell preparation including a micronized fat cell and an adipose stem cell for use in the treatment of osteoarthritis the dosage form is not specifically limited, and appropriately selected from conventionally known methods depending on the position and range in which regeneration is expected, age of the patient, and the like.

In the cell preparation including a micronized fat cell and an adipose stem cell for use in the restoration of a muscle, the method for collecting fat, the method for separating a mesodermal stem cell, the method for preparing a cell preparation, the method for administration to a patient and the like are as described in the section of "1. Cell preparation" and "2. Method for preparing a cell preparation". In the cell preparation including a micronized fat cell and an adipose stem cell for use in the treatment of osteoarthritis, the dosage form is not specifically limited, and appropriately selected from conventionally known methods depending on the position and range in which restoration of a muscle is expected, age of the patient, and the like.

The present invention provides a cell preparation including a micronized fat cell and an adipose stem cell for use in the treatment of osteoarthritis. Furthermore, the present invention provides a cell preparation including a micronized fat cell and an adiposestem cell for use in the restoration of a muscle.

### EXAMPLES

### Preparation of a cell preparation

For preparation of the cell preparation, LIPOMAX-SC (manufactured by Medikan International Inc., Seoul, Korea) kit was used. In the preparation step of the present cell preparation, it was possible to carry out the step without contacting the cells with the open air at all. The specific method is as follows.
(i) Using a syringe (a syringe manufactured for LIPOMAX-SC: manufactured by Medikan International Inc., Seoul, Korea), liposuction was carried out on each subject. A syringe containing the collected fat was left standing still at room temperature (about 25°C) for about 10 minutes to separate the fat into a cellular fraction and a liquid fraction, and the liquid fraction containing a tumescent fluid was discarded. The remaining cellular fraction was used in Test Example 2 as an adipose tissue. Thereafter, the syringe was subjected to centrifugation at 2,200 × g for 8 minutes. By centrifugation, the cellular fraction was separated into three layers of the upper layer (free oil), the intermediate layer (a fat cell and an adipose stem cell) and the lower layer (a liquid fraction such as a cellular tissue fluid). Thereafter, only the intermediate layer was left in the syringe, and the upper and lower layers were discarded, thereby a concentrate of a fat cell and an adipose stem cell was obtained. The gelling treatment was carried out for the concentrate to micronize a relatively large fat cell. The gelling treatment was carried out once to three times. A drill (Filler-Geller cutter:
   manufactured by Medikan International, Inc.) was used for gelling to cut the fat cell for micronization, and the fat cell was further centrifuged at 2,200 × g for 5 minutes, thereby a mixture including a micronized fat cell and an adipose stem cell was obtained. The mixture thus obtained was used in Test Examples 2 to 5 described below as a cell preparation including a micronized fat cell and an adipose stem cell.
(ii) Next, extraction of an adipose stem cell was carried out. To the mixture of a fat cell and an adipose stem cell obtained in step (i), a 0.4% aqueous solution of a protease (trade name: Collagenase; manufactured by Worthington Biochemical Corp.) was added in an amount same as that of the mixture, and the mixture was mildly shaken. Thereafter, the mixture was incubated at 38°C for 45 minutes.
(iii) After incubation, centrifugation was carried out at 200 × g for 5 minutes, and the supernatant was collected. A fat cell was concentrated in the supernatant, and an adipose stem cell was concentrated in the precipitate.
(iv) Next, about 50 mL of dextran (manufactured by Otsuka Pharmaceutical Co., Ltd.) was added to 2 to 3 mL of the precipitate (a concentrate of an adipose stem cell) obtained in step (iii), and the mixture was mildly shaken. Thereafter, centrifugation was carried out at 200 × g for 5 minutes, and the supernatant was discarded, thereby washing of an adipose stem cell was carried out. The procedure was repeated three times. The concentrate of an adipose stem cell obtained in the present step was used in the control group in Test Example 1 described below. In addition, the supernatant including a fat cell obtained in step (iii) was subjected to the same washing treatment, and thereafter used in Test Example 1 described below as a fat cell (and an extracellular matrix).

Furthermore, since the fat cell and the adipose stem cell derived from each patient were used, the above-mentioned procedures were carried out for each patient.

### Test Example 1. Effect of alleviating pain caused by osteoarthritis (1)

The fat cell (including an extracellular matrix) obtained in the above-mentioned step (iv) was administered to subjects having knee osteoarthritis, and change in the symptoms was observed.

The subjects were 2 women (4 cases (both subjects had a disease in both the knee joints); average age: 68.5 years old). The fat used was collected from the lower abdomen of each subject. The amount of the collected fat was 102 mL and 95 mL, respectively. A fat cell (including an extracellular matrix) was obtained from the collected fat in accordance with the above-mentioned method for preparation of a cell preparation, and 7 mL of the fat cell was injected to each of the knee joints of each subject. In addition, as a control group, hyaluronic acid (Suvenyl (manufactured by CHUGAI PHARMACEUTICAL CO., LTD.: 2.5 mL) or 7 mL of the adipose stem cell obtained in the above-mentioned step (iv) (in both subjects, the site from which the fat was collected was the lower abdomen; the amount of the collected fat was 102 mL and 95 mL, respectively) was injected to the knee joint of another 2 subjects having knee osteoarthritis. In the present Test Example 1, upon carrying out injection to the knee joint, the procedure of stimulating the site of injection by turning the tip of a cannula in all directions was not carried out.

As a result, in the subject to whom a fat cell was injected, knee joint pain was reduced from the first day of the injection, and the effect lasted for about 1 month. In addition, as compared with the subjects to whom hyaluronic acid or an adipose stem cell was injected, the effect of alleviating knee joint pain was clearly developed from an earlier stage, and the effect of alleviating pain was also remarkable.

### Test Example 2. Effect of alleviating pain caused by osteoarthritis (2)

The adipose tissue obtained in the above-mentioned step (i) was administered to subjects having knee osteoarthritis, and change in the symptoms was observed. The subjects were 3 women (6 cases (every subject had a disease in both the knee joints); average age: 69.7 years old). The fat used was collected from the lower abdomen of each subject. The amount of the collected fat was 95 mL, 100 mL and 105 mL, respectively. The adipose tissue was obtained from the collected fat in accordance with step (i) of the above-mentioned method for preparation of a cell preparation, and 7 mL of the adipose tissue was injected to each of the knee joints of each subject. In addition, as a control, hyaluronic acid (Suvenyl (manufactured by CHUGAI PHARMACEUTICAL CO., LTD.: 2.5 mL) was injected to the knee joint of another subject having knee osteoarthritis. In the present Test Example 2, upon carrying out injection to the knee joint, the procedure of stimulating the site of injection by turning the tip of a cannula in all directions was not carried out.

As a result, in any of the 3 subjects to whom an adipose tissue was injected, the effect of alleviating knee joint pain was developed earlier than in the subject to whom hyaluronic acid was injected, and the effect lasted for 1 month or more.

### Test Example 3. Effect of regeneration of a tissue on osteoarthritis

The cell preparation including a micronized fat cell and an adipose stem cell prepared in the above-mentioned step (i) (5 to 10 mL, including about 750 thousand to 2 million adipose stem cells) was injected to the knee joint of 9 women (14 cases; average age: 68.7 years old) having knee osteoarthritis, using a cannula of which tip was obliquely cut. The site of the disease, the specific site of liposuction, the amount of the collected fat, the amount of injection of the cell preparation, and presence or absence of the procedure of stimulating the site of injection by turning a cannula in all directions are shown in Table 1 below. Treatment for each subject, measurement results of the knee joint fissure and evaluation results on the basis of Kellgren and Lawrence classification which is an evaluation standard of an X-ray finding of knee osteoarthritis are summarized in Table 1 below.

**[Table 1]**

| | | Subject A | Subject B | | Subject C | Subject D | | Subject E | | Subject F | Subject G | | Subject H | | Subject I |
|---|---|---|---|---|---|---|---|---|---|---|---|---|---|---|---|
| Knee osteoarthritis | | Left | Right | Left | Right | Right | Left | Right | Left | Left | Right | I Left | Right | Left | Left |
| Site from which fat was collected | | Lower abdomen | Lower abdomen | | Lower abdomen | Femur | | Lower abdomen | | Lower abdomen | Lower abdomen | | Lower abdomen | | Femur |
| Amount of collected fat | | 50 mL | 82 mL | | 90 mL | 80 mL | | 94 mL | | 76 mL | 76 mL | | 96 mL | | 102 mL |
| Presence or absence of procedure of stimulating site of injection by cannula | | + | + | + | + | ++ | ++ | + | + | + | + | - | - | + | + |
| Amount of injection of cell preparation | | 5 mL | 7 mL | 7 mL | 8 mL | 7 mL | 8 mL | 6 mL | 7mL | 7mL | 8mL | 6mL | 6mL | 8mL | 9mL |
| Joint fissure (mm) | Before treatment | 2 | 0 | 0 | - | - | - | - | - | 1.6 | 0 | * | * | 2.4 | - |
| | After treatment | 3.3 | 2.6 | 2.6 | - | - | - | - | - | 3 | 1.8 | | | 4.4 | - |
| Kellgren and Lawrence classification (Grade) | Number of days after treatment | 3.5 weeks | 5 weeks | | 13 weeks | 12 weeks | | 7 weeks | | 11 weeks | 7.5 weeks | | 8 weeks | | 2 weeks |
| | Before treatment | IV | IV | IV | II | III | IV | II | II | IV | III | II | III | IV | - |
| | After treatment | II | II | II | I | II | III | I | I | II | II | I | II | III | - |

| | | | | | | | | | | | | | | | |
|---|---|---|---|---|---|---|---|---|---|---|---|---|---|---|---|
| * In the table, regarding knee osteoarthritis, left indicates that the subject has the disease in the left knee, and right indicates that the subject has the disease in the right knee. * In the table, regarding presence or absence of procedure of stimulating site of injection by cannula, + indicates presence of stimulation, ++ indicates presence of strong stimulation, and - indicates absence of stimulation. * In the table, regarding the size of joint fissure, - indicates that the size was not measured, and * indicates that the size remained almost unchanged after the treatment. * In the table, the number of days after treatment indicates the number of days elapsed from treatment until the Grade on the basis of Kellgren and Lawrence classification changed. * In the table, regarding Kellgren and Lawrence classification, - indicates that evaluation was not carried out since the number of days after treatment was small. | | | | | | | | | | | | | | | |

In addition, the specific evaluation items and evaluation standard of Kellgren and Lawrence classification are as follows.

**[Table 2]**

| Grade | Narrowing of joint fissure | Osteophyte formation | Consolidation | Deformation of outline of joint |
|---|---|---|---|---|
| I | Doubted | Possible | None | None |
| II | Possible | Clear | None | None |
| III | Clear | Moderate | Moderate | Possible |
| IV | Moderate | Profound | Profound | Clear |

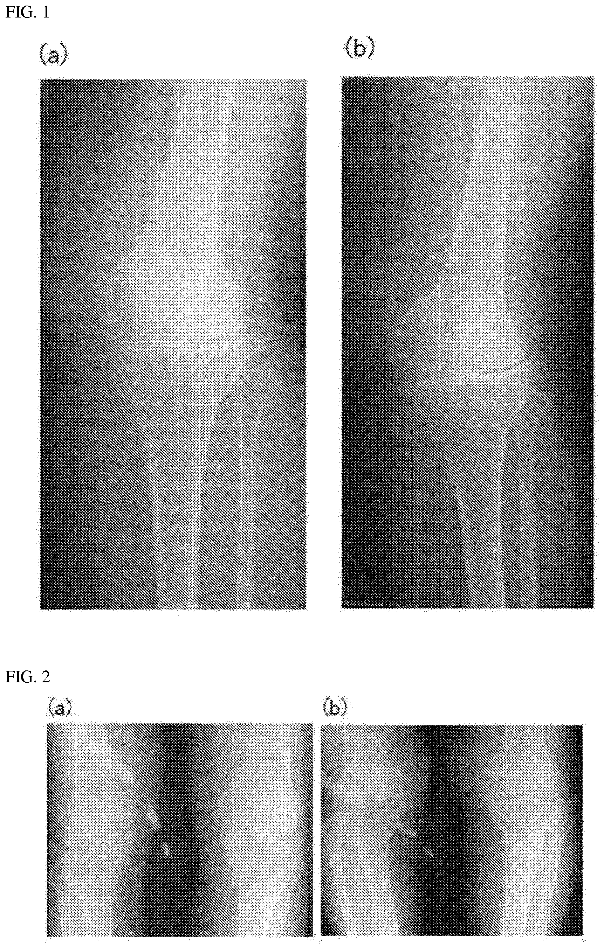

As representative exampled, X-ray photographs of the knee joints of subjects A and B are shown in Figs. 1 and 2.

### Subject A

Age at the time of treatment: 72 years old, woman
Anamnesis: knee osteoarthritis (the left knee)

To the left knee joint was injected 5 mL of the cell preparation. The X-ray photographs of the joint fissure of the outside of the left knee joint of subject A taken before injection of the cell preparation and about 1 month after injection are shown in Fig. 1.

### Subject B

Age at the time of treatment: 60 years old, woman
Anamnesis: knee osteoarthritis (both right and left knees)

To the right and left knee joints was injected 7 mL each of the cell preparation. The X-ray photographs of the joint fissure of the outside of the right and left knee joints of subject B taken before injection of the cell preparation and about 1 month after injection are shown in Fig. 2.

### (Results)

Within 5 days after the day of treatment (average: 2 days), pain in the knee joint was remarkably improved in all of the subjects, and the state of improved pain lasted even after the above-mentioned period. In addition, 2 subjects who had been walking on cane became able to walk without a cane. Furthermore, 2 subjects who had been difficult to stand up became able to stand up, and walking remarkably improved in all of the subjects.

According to the X-ray photograph finding of the representative subjects A and B, improvement in knee osteoarthritis and expansion of joint fissure were shown. That is to say, as shown in Fig. 1, while the joint fissure of the left knee joint of subject A was 2.0 m before treatment (Fig. 1(a)), the joint fissure was expanded to 3.3 mm about 1 month after treatment (Fig. 1(b)). More specifically, while the grade of Kellgren and Lawrence classification was IV before treatment, the grade was improved to II about 1 month after treatment. In addition, as shown in Fig. 2, while joint fissure of the right and left knee joints of subject B was 0 mm before treatment (Fig. 2(a)), the joint fissure was expanded to 2.6 mm about 1 month after treatment (Fig. 2(b)). More specifically, while the grade of Kellgren and Lawrence classification was IV before treatment, the grade was improved to II about 1 month after treatment.

According to the results of the present Test Example 3, it was shown that regeneration of the tissue in the knee joint was enhanced by injecting the cell preparation including a micronized fat cell and an adipose stem cell to the knee joint of the subject having knee osteoarthritis. On the other hand, according to the X-ray photograph finding of the subjects to whom an adipose stem cell was injected as a control group of the above-mentioned Test Example 1, although regeneration of a tissue was found, speed of advance of regeneration of a tissue and improvement of the symptom were clearly slower than in subjects A and B.

### Test Example 4. Effect of regeneration of tissue for osteonecrosis

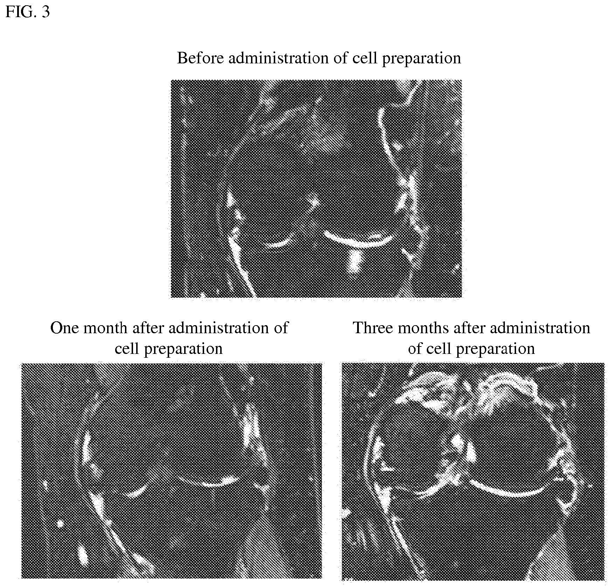

The cell preparation including a micronized fat cell and an adipose stem cell prepared in the above-mentioned step (i) (5 to 10 mL, including about 750 thousand to 2 million adipose stem cells) was injected to the site of osteonecrosis of 3 subjects of subjects A, B and J in Test Example 3 who additionally developed osteonecrosis in combination with knee osteoarthritis. Regarding subject J, the site of the disease, the specific site of liposuction, the amount of the collected fat and the amount of injection of the cell preparation are shown in Table 3 below. In addition, an MR image of the knee joint of subject A is shown in Fig. 3 as a representative example.

**[Table 3]**

| | | Subject J | |
|---|---|---|---|
| Knee osteoarthritis | | Right | Left |
| Site from which fat was collected | | Lower abdomen, both femurs | |
| Amount of collected fat | | 212 mL | |
| Presence or absence of procedure of stimulating site of injection by cannula | | + | + |
| Amount of injection of cell preparation | | 10 mL | 9 mL |
| Presence or absence of image of osteonecrosis before treatment | | Present | Present |
| Kellgren and Lawrence classification (Grade) | Number of days after treatment | 12 weeks | 12 weeks |
| | Before treatment | IV | IV |
| | After treatment | III | III |

In any of the subjects, symptoms such as walking difficulty and pain were remarkably improved within 2 days after injection of the cell preparation into the joint. In addition, regarding subject J, as shown in Table 3, while the grade of Kellgren and Lawrence classification was IV before treatment, the symptom was improved to III about 3 months after treatment. Furthermore, as shown in the MR image of the knee joint of subject A (Fig. 3), remarkable improvement in the image of osteonecrosis was found 1 month after injection of the cell preparation into the joint, and osteonecrosis was disappeared 3 months after the injection. When MR images in regard to the knee joints of subjects A and B were taken, improvement or disappearance of images of osteonecrosis and bone edema was found 1 to 3 months after injection of the cell preparation in either of the subjects.

### Test Example 5. Effect of restoration of a muscle

The cell preparation including a micronized fat cell and an adipose stem cell prepared in the above-mentioned step (i) (10 to 50 mL, including about 4 million to 20 million adipose stem cells) was injected to the site of muscle damage of subjects having damage in quadriceps femoris muscle, damage in deltoid muscle of the upper arm or damage in pectoralis major (4 men; average age: 37.8 years old, 20 women; average age: 33.8 years old). For preparation of the cell preparation, fat collected from the lower abdomen or the femur of the subjects (the amount of collected fat: 50 to 200 mL) was used.

Regarding improvement of pain caused by muscle damage after injection of the cell preparation, the results were as follows: very little pain has been felt since injection (8 subjects: women); little pain has been felt since injection (4 subjects: men); pain disappeared 1 day after injection (10 subjects: women); and pain disappeared 2 to 3 days after injection (2 subjects: women). That is to say, it was shown that pain due to muscle damage is improved from an early stage by injection of a cell preparation including a fat cell and an adipose stem cell.

In addition, the range of motion (ROM: Range of Motion) of the joint of the upper limbs was expanded in all of the subjects within 3 weeks to 1 month after injection of the cell preparation, and improvement of motor function was found. That is to say, it was shown that a muscle is restored by administering the cell preparation to the site of muscle damage.

### 3. Conclusion

As described above, it was shown that pain caused by osteoarthritis can be early alleviated by injecting a fat cell to the affected part of a patient of knee osteoarthritis, and moreover that the effect lasts for a long time (Test Example 1). In addition, when an adipose tissue was injected, pain was early alleviated as in the case of Test Example 1, and the effect lasted for a still longer time (Test Example 2). Furthermore, by injecting a cell preparation including a micronized fat cell and a mesodermal stem cell (an adipose stem cell) in a high concentration to the affected part, regeneration of the tissue of the affected part was enhanced, in addition to the above-mentioned effect of alleviating pain (Test Example 3). Moreover, comparing the case where only an adipose stem cell was injected (control group of Test Example 1) with the case where the cell preparation including a micronized fat cell and an adipose stem cell was injected (Test Example 3), the effect of regeneration of a tissue was synergistically augmented when the cell preparation including a micronized fat cell and an adipose stem cell was injected. In addition, when the cell preparation including a micronized fat cell and an adipose stem cell was injected to the site of osteonecrosis (Test Example 4), regeneration of a bone tissue in the part of necrosis was found. Furthermore, it was shown that a muscle is restored by administering a fat cell and a mesodermal stem cell to the site of muscle damage (Test Example 5). It can be thought that, when a fat cell and a mesodermal stem cell such as an adipose stem cell are used in combination, the mesodermal stem cell is retained in the affected part by the action of the fat cell (including an extracellular matrix), thereby the efficiency of engraftment of a mesodermal stem cell and the efficiency of regeneration of a tissue are augmented.

According to the test examples described above, it was shown that the cell preparation of the present invention exhibits an excellent effect in treatment of osteoarthritis and restoration of a muscle.

## Claims

1. A cell preparation for use in the treatment of osteoarthritis, comprising:
a mixture including a micronized fat cell and an adipose stem cell, the micronized fat cell and the adipose stem cell being obtained by steps of:
separating an adipose tissue taken from a subject into a cellular fraction and a liquid fraction to obtain the cellular fraction;
removing impurities from the cellular fraction to obtain a concentrate of a fat cell and an adipose stem cell; and
micronizing the fat cell in the concentrate of the fat cell and the adipose stem cell, wherein there are 100,000 or more adipose stem cells per 1 mL of the cell preparation.

2. A cell preparation for use in the treatment of osteoarthritis, comprising:
a mixture including a micronized fat cell and an adipose stem cell, wherein the micronized fat cell and the adipose stem cell are obtained by steps of:
separating an adipose tissue taken from a subject into a cellular fraction and a liquid fraction to obtain the cellular fraction;
removing impurities from the cellular fraction to obtain a concentrate of a fat cell and an adipose stem cell;
micronizing the fat cell in the concentrate of the fat cell and the adipose stem cell to obtain a mixture including the micronized fat cell and the adipose stem cell;
separating the adipose stem cell from the mixture of the micronized fat cell and the adipose stem cell to obtain the adipose stem cell; and
mixing the separated adipose stem cell with the concentrate of the micronized fat cell and the adipose stem cell, wherein there are 100,000 or more adipose stem cells per 1 mL of the cell preparation.

3. The cell preparation for use in the treatment of osteoarthritis according to claim 1 or 2, comprising 1 to 10 adipose stem cells per one fat cell.

4. A method for manufacturing a cell preparation for use in the treatment of osteoarthritis, comprising steps of:
separating an adipose tissue taken from a subject into a cellular fraction and a liquid fraction to obtain the cellular fraction;
removing impurities from the cellular fraction to obtain a concentrate of a fat cell and an adipose stem cell; and
micronizing the fat cell in the concentrate of the fat cell and the adipose stem cell to obtain a mixture including the micronized fat cell and the adipose stem cell, wherein
the cell preparation comprises a mixture including a micronized fat cell and an adipose stem cell, wherein there are 100,000 or more adipose stem cells per 1 mL of the cell preparation.

5. A method for manufacturing a cell preparation for use in the treatment of osteoarthritis, comprising steps of:
separating an adipose tissue taken from a subject into a cellular fraction and a liquid fraction to obtain the cellular fraction;
removing impurities from the cellular fraction to obtain a concentrate of a fat cell and an adipose stem cell;
micronizing the fat cell in the concentrate of the fat cell and the adipose stem cell to obtain a mixture including the micronized fat cell and the adipose stem cell;
separating the adipose stem cell from the mixture including the micronized fat cell and the adipose stem cell to obtain the adipose stem cell; and
mixing the separated adipose stem cell with remainder of the concentrate of the micronized fat cell and the adipose stem cell to obtain the mixture including the micronized fat cell and the adipose stem cell, wherein
the cell preparation comprises a mixture including a micronized fat cell and an adipose stem cell, wherein there are 100,000 or more adipose stem cells per 1 mL of the cell preparation.

6. The method for manufacturing the cell preparation for use in the treatment of osteoarthritis according to claim 4 or 5, wherein the cell preparation comprises 1 to 10 adipose stem cells per one fat cell.

7. A cell preparation for use in the restoration of a muscle, comprising a mixture including a micronized fat cell and an adipose stem cell, wherein the micronized fat cell and the adipose step cell are obtained by the steps of:
separating an adipose tissue taken from a subject into a cellular fraction and a liquid fraction to obtain the cellular fraction;
removing impurities from the cellular fraction to obtain a concentrate of a fat cell and an adipose stem cell; and
micronizing the fat cell in the concentrate of the fat cell and the adipose stem cell, wherein there are 100,000 or more adipose stem cells per 1 mL of the cell preparation.

8. A method for manufacturing a cell preparation for use in the restoration of a muscle, the method comprising steps of:
separating an adipose tissue taken from a subject into a cellular fraction and a liquid fraction to obtain the cellular fraction;
removing impurities from the cellular fraction to obtain a concentrate of a fat cell and an adipose stem cell; and
micronizing the fat cell in the concentrate of the fat cell and the adipose stem cell to obtain a mixture including the micronized fat cell and the adipose stem cell, wherein
the cell preparation comprises the mixture including the micronized fat cell and the adipose stem cell, wherein there are 100,000 or more adipose stem cells per 1 mL of the cell preparation.

## Patentansprüche

1. Zellpräparat zur Verwendung zur Behandlung von Osteoarthritis, das Folgendes umfasst:
ein Gemisch, das mikronisierte Fettzellen und Fettstammzellen umfasst, wobei die mikronisierten Fettzellen und die Fettstammzellen durch folgende Schritte erhalten werden:
das Trennen von Fettgewebe, das einem Individuum abgenommen wurde, in eine Zellfraktion und eine Flüssigkeitsfraktion zum Erhalt der Zellfraktion;
das Entfernen von Verunreinigungen aus der Zellfraktion zum Erhalt eines Konzentrats der Fettzellen und der Fettstammzellen; und
das Mikronisieren der Fettzellen in dem Konzentrat der Fettzellen und der Fettstammzellen, wobei 100.000 oder mehr Fettstammzellen pro 1 ml des Zellpräparats vorliegen.

2. Zellpräparat zur Verwendung zur Behandlung von Osteoarthritis, das Folgendes umfasst:
ein Gemisch, das mikronisierte Fettzellen und Fettstammzellen umfasst, wobei die mikronisierte Fettzellen und die Fettstammzellen durch folgende Schritte erhalten werden:
das Trennen von Fettgewebe, das einem Individuum abgenommen wurde, in eine Zellfraktion und eine Flüssigkeitsfraktion zum Erhalt der Zellfraktion;
das Entfernen von Verunreinigungen aus der Zellfraktion zum Erhalt eines Konzentrats der Fettzellen und der Fettstammzellen; und
das Mikronisieren der Fettzellen in dem Konzentrat der Fettzellen und der Fettstammzellen, um ein Gemisch zu erhalten, das die mikronisierten Fettzellen und die Fettstammzellen umfasst;
das Abtrennen der Fettstammzellen von dem Gemisch aus den mikronisierten Fettzellen und den Fettstammzellen zum Erhalt der Fettstammzellen; und
das Vermischen der abgetrennten Fettstammzellen mit dem Konzentrat der mikronisierten Fettzellen und der Fettstammzellen, wobei 100.000 oder mehr Fettstammzellen pro 1 ml des Zellpräparats vorliegen.

3. Zellpräparat zur Verwendung zur Behandlung von Osteoarthritis nach Anspruch 1 oder 2, das 1 bis 10 Fettstammzellen pro Fettzelle umfasst.

4. Verfahren zur Herstellung eines Zellpräparats zur Verwendung zur Behandlung von Osteoarthritis, wobei das Verfahren die folgenden Schritte umfasst:
das Trennen von Fettgewebe, das einem Individuum abgenommen wurde, in eine Zellfraktion und eine Flüssigkeitsfraktion zum Erhalt der Zellfraktion;
das Entfernen von Verunreinigungen aus der Zellfraktion zum Erhalt eines Konzentrats von Fettzellen und Fettstammzellen; und
das Mikronisieren der Fettzellen in dem Konzentrat der Fettzellen und der Fettstammzellen, um ein Gemisch zu erhalten, das die mikronisierten Fettzellen und die Fettstammzellen umfasst,
wobei das Zellpräparat ein Gemisch umfasst, das mikronisierte Fettzellen und Fettstammzellen umfasst, wobei 100.000 oder mehr Fettstammzellen pro 1 ml des Zellpräparats vorliegen.

5. Verfahren zur Herstellung eines Zellpräparats zur Verwendung zur Behandlung von Osteoarthritis, wobei das Verfahren die folgenden Schritte umfasst:
das Trennen von Fettgewebe, das einem Individuum abgenommen wurde, in eine Zellfraktion und eine Flüssigkeitsfraktion zum Erhalt der Zellfraktion;
das Entfernen von Verunreinigungen aus der Zellfraktion zum Erhalt eines Konzentrats von Fettzellen und Fettstammzellen; und
das Mikronisieren der Fettzellen in dem Konzentrat der Fettzellen und der Fettstammzellen, um ein Gemisch zu erhalten, das die mikronisierten Fettzellen und die Fettstammzellen umfasst;
das Abtrennen der Fettstammzellen von dem Gemisch aus den mikronisierten Fettzellen und den Fettstammzellen zum Erhalt der Fettstammzellen; und
das Vermischen der abgetrennten Fettstammzellen mit dem Rest des Konzentrats der mikronisierten Fettzellen und Fettstammzellen zum Erhalt eines Gemischs, das die mikronisierten Fettzellen und die Fettstammzellen umfasst, wobei
das Zellpräparat ein Gemisch umfasst, das mikronisierte Fettzellen und Fettstammzellen umfasst, wobei 100.000 oder mehr Fettstammzellen pro 1 ml des Zellpräparats vorliegen.

6. Verfahren zur Herstellung eines Zellpräparats zur Verwendung zur Behandlung von Osteoarthritis nach Anspruch 4 oder 5, wobei das Zellpräparat 1 bis 10 Fettstammzellen pro Fettzelle umfasst.

7. Zellpräparat zur Verwendung zur Wiederherstellung von Muskeln, das ein Gemisch umfasst, das mikronisierte Fettzellen und Fettstammzellen umfasst, wobei die mikronisierten Fettzellen und die Fettstammzellen durch die folgenden Schritte erhalten werden:
das Trennen von Fettgewebe, das einem Individuum Abgenommen wurde, in eine Zellfraktion und eine Flüssigkeitsfraktion zum Erhalt der Zellfraktion;
das Entfernen von Verunreinigungen aus der Zellfraktion zum Erhalt eines Konzentrats der Fettzellen und der Fettstammzellen; und
das Mikronisieren der Fettzellen in dem Konzentrat der Fettzellen und der Fettstammzelle, wobei 100.000 oder mehr Fettstammzellen pro 1 ml des Zellpräparats vorliegen.

8. Verfahren zur Herstellung eines Zellpräparats zur Verwendung zur Wiederherstellung von Muskeln, wobei das Verfahren die folgenden Schritte umfasst:
das Trennen von Fettgewebe, das einem Individuum abgenommen wurde, in eine Zellfraktion und eine Flüssigkeitsfraktion zum Erhalt der Zellfraktion;
das Entfernen von Verunreinigungen aus der Zellfraktion zum Erhalt eines Konzentrats von Fettzellen und Fettstammzellen; und
das Mikronisieren der Fettzellen in dem Konzentrat der Fettzellen und der Fettstammzellen zum Erhalt eines Gemischs, dass die mikronisierten Fettzellen und die Fettstammzellen umfasst,
wobei das Zellpräparat das Gemisch umfasst, das die mikronisierten Fettzellen und die Fettstammzellen umfasst, wobei 100.000 oder mehr Fettstammzellen pro 1 ml des Zellpräparats vorliegen.

## Revendications

1. Préparation cellulaire pour une utilisation dans le traitement de l'arthrose, comprenant :
un mélange incluant une cellule grasse micronisée et une cellule souche adipeuse, la cellule grasse micronisée et la cellule souche adipeuse étant obtenues par les étapes de :
séparation d'un tissu adipeux prélevé auprès d'un sujet en une fraction cellulaire et une fraction liquide pour obtenir la fraction cellulaire ;
élimination des impuretés de la fraction cellulaire pour obtenir un concentré d'une cellule grasse et d'une cellule souche adipeuse ; et
micronisation de la cellule grasse dans le concentré de la cellule grasse et de la cellule souche adipeuse, dans laquelle il y a 100 000 cellules souches adipeuses ou plus pour 1 ml de préparation cellulaire.

2. Préparation cellulaire pour une utilisation dans le traitement de l'arthrose, comprenant :
un mélange incluant une cellule grasse micronisée et une cellule souche adipeuse, la cellule grasse micronisée et la cellule souche adipeuse étant obtenues par les étapes de :
séparation d'un tissu adipeux prélevé auprès d'un sujet en une fraction cellulaire et une fraction liquide pour obtenir la fraction cellulaire ;
élimination des impuretés de la fraction cellulaire pour obtenir un concentré d'une cellule grasse et d'une cellule souche adipeuse ;
micronisation de la cellule grasse dans le concentré de la cellule grasse et de la cellule souche adipeuse pour obtenir un mélange incluant la cellule grasse micronisée et la cellule souche adipeuse ;
séparation de la cellule souche adipeuse du mélange de la cellule grasse et de la cellule souche adipeuse pour obtenir la cellule souche adipeuse ; et
mélange de la cellule source adipeuse séparée avec le concentré de la cellule grasse et de la cellule souche adipeuse, dans lequel il y a 100 000 cellules souches adipeuses ou plus pour 1 ml de préparation cellulaire.

3. Préparation cellulaire pour une utilisation dans le traitement de l'arthrose selon la revendication 1 ou 2, comprenant de 1 à 10 cellules souches adipeuses pour une cellule grasse.

4. Procédé de fabrication d'une préparation cellulaire pour une utilisation dans le traitement de l'arthrose, comprenant les étapes de :
séparation d'un tissu adipeux prélevé auprès d'un sujet en une fraction cellulaire et une fraction liquide pour obtenir la fraction cellulaire ;
élimination des impuretés de la fraction cellulaire pour obtenir un concentré d'une cellule grasse et d'une cellule souche adipeuse ; et
micronisation de la cellule grasse dans le concentré de la cellule grasse et de la cellule souche adipeuse pour obtenir un mélange incluant la cellule grasse micronisée et la cellule souche adipeuse, dans lequel
la préparation cellulaire comprend un mélange incluant une cellule grasse micronisée et une cellule souche adipeuse, dans lequel il y a 100 000 cellules souches adipeuses ou plus pour 1 ml de préparation cellulaire.

5. Procédé de fabrication d'une préparation cellulaire pour une utilisation dans le traitement de l'arthrose, comprenant les étapes de :
séparation d'un tissu adipeux prélevé auprès d'un sujet en une fraction cellulaire et une fraction liquide pour obtenir la fraction cellulaire ;
élimination des impuretés de la fraction cellulaire pour obtenir un concentré d'une cellule grasse et d'une cellule souche adipeuse ; et
micronisation de la cellule grasse dans le concentré de la cellule grasse et de la cellule souche adipeuse pour obtenir un mélange incluant la cellule grasse micronisée et la cellule souche adipeuse ;
séparation de la cellule souche adipeuse du mélange incluant la cellule grasse et la cellule souche adipeuse pour obtenir la cellule souche adipeuse ; et
mélange de la cellule source adipeuse séparée avec le reste du concentré de la cellule grasse micronisée et de la cellule souche adipeuse pour obtenir un mélange incluant la cellule grasse micronisée et la cellule souche adipeuse, dans lequel
la préparation cellulaire comprend un mélange incluant une cellule grasse micronisée et une cellule souche adipeuse, dans lequel il y a 100 000 cellules souches adipeuses ou plus pour 1 ml de préparation cellulaire.

6. Procédé de fabrication de la préparation cellulaire pour une utilisation dans le traitement de l'arthrose selon la revendication 4 ou 5, dans lequel la préparation cellulaire comprend de 1 à 10 cellules souches adipeuses par cellule grasse.

7. Préparation cellulaire pour une utilisation dans la régénération d'un muscle, comprenant un mélange incluant une cellule grasse micronisée et une cellule souche adipeuse, la cellule grasse micronisée et la cellule souche adipeuse étant obtenues par les étapes de :
séparation d'un tissu adipeux prélevé auprès d'un sujet en une fraction cellulaire et une fraction liquide pour obtenir la fraction cellulaire ;
élimination des impuretés de la fraction cellulaire pour obtenir un concentré d'une cellule grasse et d'une cellule souche adipeuse ; et
micronisation de la cellule grasse dans le concentré de la cellule grasse et de la cellule souche adipeuse, dans laquelle il y a 100 000 cellules souches adipeuses ou plus pour 1 ml de préparation cellulaire.

8. Procédé de fabrication d'une préparation cellulaire pour une utilisation dans la régénération d'un muscle, le procédé comprenant les étapes de :
séparation d'un tissu adipeux prélevé auprès d'un sujet en une fraction cellulaire et une fraction liquide pour obtenir la fraction cellulaire ;
élimination des impuretés de la fraction cellulaire pour obtenir un concentré d'une cellule grasse et d'une cellule souche adipeuse ; et
micronisation de la cellule grasse dans le concentré de la cellule grasse et de la cellule souche adipeuse pour obtenir un mélange incluant la cellule grasse micronisée et la cellule souche adipeuse, dans lequel
la préparation cellulaire comprend le mélange incluant la cellule grasse micronisée et la cellule souche adipeuse, dans lequel il y a 100 000 cellules souches adipeuses ou plus pour 1 ml de préparation cellulaire.
